(19) Europäisches Patentamt
European Patent Office
Office européen des brevets

(11) Publication number : **0 485 091 A1**

(12) **EUROPEAN PATENT APPLICATION**

(21) Application number : **91309916.4**

(22) Date of filing : **28.10.91**

(51) Int. Cl.$^5$ : **C07H 19/01,** C12N 1/20, C12P 19/26, // (C12P19/26, C12R1:55), (C12N1/20, C12R1:55)

The applicant has filed a statement in accordance with Rule 28 (4) EPC (issue of a sample only to an expert). Accessionnumber(s) of the deposit(s): ATCC 14891 and ATCC 55087.

(30) Priority : **29.10.90 US 605598**

(43) Date of publication of application : **13.05.92 Bulletin 92/20**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Byrne, Kevin M.**
**6 Forest Lane**
**West Trenton, NJ 08628 (US)**
Inventor : **Kaplan, Louis**
**7 Lexington Road**
**New City, NY 10956 (US)**
Inventor : **Arison, Byron H.**
**88 Century Lane**
**Watchung, NJ 07060 (US)**
Inventor : **Colwell, Lawrence F., Jr.**
**5 Princess Lane**
**Eatontown, NJ 07724 (US)**

(74) Representative : **Thompson, John Dr. et al**
**Merck & Co., Inc. European Patent Department Terlings Park Eastwick Road**
**Harlow, Essex CM20 2QR (GB)**

(54) **Process for the production of analogues of immunomycin.**

(57) This invention relates to a novel process for producing immunomycin and a C-21 homolog thereof, these compounds having immunosuppressant activity. This process involves directed biosynthesis, a fermentation process in which the novel cultured organism is grown in the presence of a higher analog of butyric acid.

EP 0 485 091 A1

## SUMMARY OF THE INVENTION

The present invention is a fermentation process consisting of the supplemental feeding of a mutant strain (Merck No. MA 6678, ATCC No. 55087) of Streptomyces hygroscopicus subsp. ascomyceticus (ATCC No. 14891), with valeric acid. The mutant strain (Merck No. MA 6678, ATCC No. 55087) without direction, has been found to produce increased quantities of immunomycin and its lower C-21 homolog, compound B. The novel fermentation process of this invention is referred to as directed biosynthesis and it is an effective method of producing a C-21 propyl analog (compound C), when the culture is supplemented with a higher homolog of butyric acid, such as valeric acid.

Immunomycin and its analogs are described in the following formula.

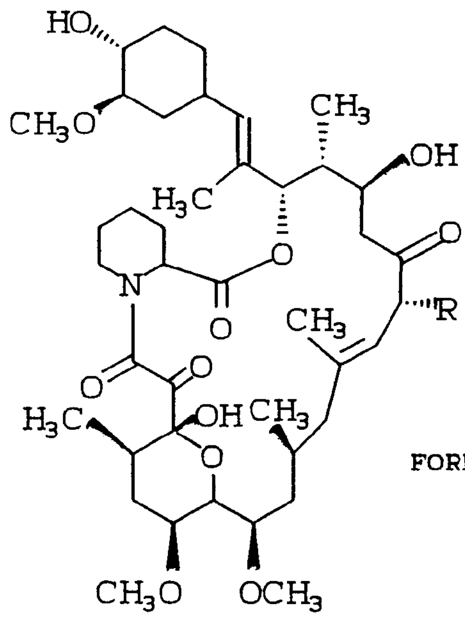

FORMULA I

| | |
|---|---|
| Compound A | $R=CH_2CH=CH_2$ |
| IMMUNOMYCIN | $R=CH_2CH_3$ |
| Compound B | $R=CH_3$ |
| Compound C | $R=CH_2CH_2CH_3$ |

The natural biosynthetic pathway utilizes butyric acid or a biologically activated form of butyric acid as a precursor for carbon atoms 20, 21, 35 and 36 in the synthesis of immunomycin, where R is ethyl in Formula I. The organism (Merck No. MA6678, ATCC No. 55087) has incorporated the unnatural substrate a homolog of butyric acid, directing the biosynthesis of the the higher C-21 homolog of immunomycin, in addition to the natural products.

## BACKGROUND OF THE INVENTION

Immunomycin is a fermentation product of the Streptomyces hygroscopicus subsp. ascomyceticus culture

(ATCC 14891). Immunomycin has activity superior to cyclosporin, which at the present time is the sole FDA approved anti-rejection drug used in the field of organ transplants. The immunosuppressant activity is also useful as a method of treatment of autoimmune diseases, as well as infectious diseases. The serious side effects associated with cyclosporin treatment include kidney failure, liver damage, and ulcers, and has prompted the development of alternate treatments. The current goal in this field of research is to develop a safer and a more effective method of treatment.

A mutant strain (Merck No. MA6678, ATCC No. 55087) of Streptomyces hygroscopicus subsp. ascomyceticus (ATCC No. 14891) surprisingly has now been shown to produce increased amounts of immunomycin and its higher homolog at C-21 by a directed biosynthesis.

A recently filed application, US Ser. No. 142,620, corresponding to EP-A-0323865, describes a new method of producing immunosuppressant, immunomycin and the immunosuppressant activity of ascomycin.

## DETAILED DECRIPTION OF THE INVENTION OF PREFERRED EMBODIMENTS

The novel process of this invention comprises the fermentative production of a compound of Formula I.

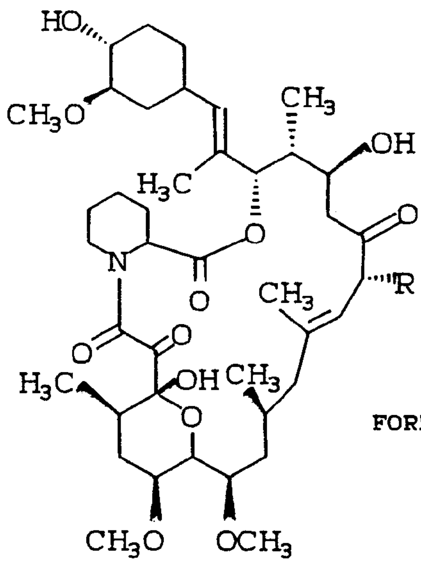

FORMULA I

wherein

R is propyl,

by fermentation of Streptomyces hygroscopicus subsp. ascomyceticus (ATCC No. 14891) or a mutant strain thereof in a nutrient medium, in the presence of valeric acid or a salt thereof as a directing component.

The following is a general description of Streptomyces hygroscopicus subsp. ascomyceticus strain MA6678, ATCC 55087 and the parent culture MA6475, ATCC 14891. Observations of growth, general cultural characteristics and carbon source utilization were made in accordance with the methods of Shirling and Gottleib (Internat. J. System. Bacteriol. 16: 313 - 340). Chemical composition of the cells was determined using the methods of Lechevalier and Lechevalier (in Actinomycete Taxonomy, A. Dietz and D.W. Thayer, Ed. Society for Industrial Microbiology, 1980). Coloration of the culture was determined by comparison with color standards Centroid Color Charts (US Dept. of Commerce National Bureau of Standards Centroid Color Charts (US Dept. of Commerce National Bureau of Standards supplement to NBS Circular 553, 1985).

**Source** -

Strain MA6678 (ATCC 55087) was derived from MA6475 (ATCC 14891), Streptomyces hygroscopicus subsp ascomyceticus.

**Analysis of Cell Wall Composition** -

The peptidoglycan of strains MA6678 and MA6475 both contain L-diaminopimelic acid.

**General growth characteristics** -

Both cultures are found to grow well on Yeast Malt Extract, Glycerol Asparagine, Inorganic Salts-Starch, Oatmeal, and Trypticase Soy agars. Growth occurs at 27°C and 37°C. Both cultures also grow well in liquid media such as Yeast Dextrose broth.

**Colony morphology** (on Yeast Malt Extract Agar)

MA6678 - Substrate mycelium is brilliant yellow (83 brill Y) and colonies are opaque, raised, erose and rubbery. The colony surface is powdery to rough textured. Aerial mycelia appear after 7 days incubation and are white in color (263 White). Spore mass, when present, is white (263 White) and turns to black (267 Black) upon prolonged incubation.

MA6475 - Substrate mycelium is medium yellow (87 m.Y) and colonies are opaque, raised, erose and rubbery. The colony surface is powdery. Aerial mycelia appear by 14 days and are medium gray (265 med Gray). Spore mass, when present is medium gray (265 med Gray) and turns black (267 Black) upon prolonged incubation.

**Micromorphology MA6678 and MA6475**

Aerial mycelium (0.57 - 0.76 μm dia.) arises from a substrate mycelium and is branched and flexous. In mature cultures, the aerial mycelium commonly terminates in spores borne predominantly in spiral chains. Spore mass tends to coalesce into an amorphous mass by 21 days.

Yeast Extract-Malt Extract

| | MA6475 | MA6678 |
|---|---|---|
| Amount of Growth: | Good | Good |
| Aerial Mycelium: | Medium gray (265 med. Gray) branched spiral sporophores | White (263 White) branched, spiral sporophores |
| Soluble Pigments: | None | None |
| Substrate Mycelium: | Medium yellow (87 m.Y) | Brilliant yellow (83 brill Y) |

Glucose Asparagine

| | MA6475 | MA6678 |
|---|---|---|
| Amount of Growth: | Good | sparse |
| Aerial Mycelium: | Medium gray (265 med. Gray) branched spiral sporophores | Yellow white (92 y. White) branched, spiral sporophores |
| Soluble Pigments: | None | None |
| Substrate Mycelium: | Pale yellow (89 p.Y) | Light yellow (86 l.Y) |

Inorganic Salts Starch

| | MA6475 | MA6678 |
|---|---|---|
| Amount of Growth: | Good | Good |
| Aerial Mycelium: | Medium gray (265 med. Gray) branched spiral sporophores | Yellow white (92 y. White) branched, spiral sporophores |
| Soluble Pigments: | None | None |
| Substrate Mycelium: | Pale yellow (89 p.Y) | Pale yellow (89 p.Y) |

Oatmeal

| | MA6475 | MA6678 |
|---|---|---|
| Amount of Growth: | Good | Good |
| Aerial Mycelium: | Dark gray (265 d. Gray) branched spiral sporophores | White (263 White) branched, spiral sporophores |
| Soluble Pigments: | None | None |
| Substrate Mycelium: | Gray yellow (90 gy.Y) | Pale yellow (89 p.Y) |

Sigma Water

| | MA6475 | MA6678 |
|---|---|---|
| Amount of Growth: | Fair | Sparse |
| Aerial Mycelium: | Black (267 Black) edges, spiral sporophores | ---- |
| Soluble Pigments: | None | None |
| Substrate Mycelium: | ---- | ---- |

Czpek

| | MA6475 | MA6678 |
|---|---|---|
| Amount of Growth: | Good | Good |
| Aerial Mycelium: | Light gray (264 l. Gray) branched spiral sporophores | White (263 White) branched, spiral sporophores |
| Soluble Pigments: | None | None |
| Substrate Mycelium: | Medium gray (265 med Gy) | Yellow white (92 y White) |

Peptone Iron

| | MA6475 | MA6678 |
|---|---|---|
| Amount of Growth: | Good | Good |
| Aerial Mycelium: | ---- | ---- |
| Soluble Pigments: | melanin negative | melanin negative |
| Substrate Mycelium: | ---- | ---- |

**Miscellaneous physiological reactions**

MA6678 - Culture does not produce melanoid pigments in tryptone yeast extract broth, starch is hydrolyzed, yellow, non-pH dependant diffusible pigment produced on Pridham-Gottlieb basal medium supplemented with 1% cellobiose, D-fructose or D-mannose. Carbon source utilization pattern is as follows: good utilization of cellobiose, D-fructose, α-D-lactose, β-D-lactose, D-maltose, D-mannitol, D-mannose, or L-rhamnose; poor utilization of L-arabinose; no utilization of D-arabinose, inositol, D-raffinose, sucrose, D-xylose, or L-xylose.

MA6475 - Culture does not produce melanoid pigments in tryptone yeast extract broth, starch is hydrolyzed. Carbon source utilization pattern is as follows: good utilization of cellobiose, D-fructose, α-D-lactose, β-D-lactose, D-maltose, D-mannitol, D-mannose, L-rhamnose or D-xylose; poor utilization of L-arabinose or inositol; no utilization of D-arabinose, D-raffinose, sucrose, or L-xylose.

**Diagnosis** -

The chemotaxonomic and morphological characteristics of strain MA6678 compare favorably with those of the parent strain MA6475. Phenotypic characteristics which serve to differentiate the MA6678 from the parent strain include: the production of a soluble yellow pigment on some media and the failure to utilize inositol or D-xylose as a sole carbon source. It should be pointed out that the specific epithet Streptomyces hygroscopicus (and hence all subspecies) is currently considered to be a subjective synonym of Streptomyces violaceusniger (Bergey's Manual of Systematic Bacteriology, Vol 4, 1989).

| Carbon Source | Utilization by MA6475 | Utilization by MA6678 |
|---|---|---|
| D-arabinose | 0 | 0 |
| L-arabinose | 1 | 1 |
| cellobiose | 2 | 2 |
| D-fructose | 2 | 2 |
| inositol | 1 | 0 |
| α-D-lactose | 2 | 2 |
| β-D-lactose | 2 | 2 |
| D-maltose | 2 | 2 |
| D-mannitol | 2 | 2 |
| D-mannose | 2 | 2 |
| D-raffinose | 0 | 0 |
| L-rhamnose | 2 | 2 |
| sucrose | 0 | 0 |
| D-xylose | 2 | 0 |
| L-xylose | 0 | 0 |
| a-D-glucose (control) | 2 | 3 |

3 = good utilization, 2 = moderate utilization, 1 = poor utilization 0 = no utilization

The fermentation is usually conducted at a temperature between about 20°C and 40°C, preferably 25-35°C, for a period of about 50 hours to 150 hours, which may be varied according to fermentation conditions and scales. Preferably, the production cultures are incubated for about 96 hours at 27°C on a rotary shaker operating at 220 rpm, wherein the pH of the fermentation medium is maintained at below 8.0 to harvest.

To the fermentation mixture, during the first 54 hrs of growth, the fatty acid primer or a related substrate thereof, for the purpose of directing the biosynthesis, was added either in one addition or multiple additions to give a final concentration of the substrate of 10-30 mm. The fermentations were harvested at 72 or 96 hours or 18-42 hours after the addition.

The conditions for the production of immunomycin and its analogs in a large quantity, preferrably employ submerged aerobic culture conditions. For the production in a small quantity, a shaking or surface culture in a flask or bottle is employed. Furthermore, when the growth is carried out in large tanks, it is preferable to use the vegetative form of the organism for inoculation in the production tanks in order to avoid growth lag in the process of production of analogs. Accordingly, it is desirable first to produce a vegetative inoculum of the organism by inoculating a relatively small quantity of culture medium with spores or mycelia of the organism produced in a "slant" and culturing said inoculated medium, also called the "seed medium", and then to transfer the cultured vegetative inoculum aseptically to large tanks. The fermentation medium, in which the vegetative inoculum is produced, is similar to the medium utilized for the production of analogs of immunomycin and is generally autoclaved to sterilize the medium prior to inoculation. The pH of the medium is generally adjusted to below 8.0 prior to the autoclaving step by suitable addition of an acid or base, preferably in the form of a buffering solution.

Agitation and aeration of the culture mixture may be accomplished in a variety of ways. Agitation may be provided by a propeller or similar mechanical agitation equipment, by revolving or shaking the fermentor, by various pumping equipment or by the passage of sterile air through the medium Aeration may be effected by

passing sterile air through the fermentation mixture.

Immunomycin and its lower C-21 homologs can be produced by culturing (fermenting) the immunomycin substance-producing strain in an aqueous nutrient medium containing sources of assimilable carbon and nitrogen, preferably under submerged aerobic conditions (e.g. shaking culture, submerged culture, etc.). The aqueous medium must be maintained below pH 8.0 at the initiation and termination (harvest) of the fermentation process. A higher pH leads to substantial and/or total loss of product. The desired pH may be maintained by the use of a buffer such as morpholinoethanesulfonic acid (MES), morpholinopropanesulfonic acid (MOPS), and the like, or by choice of nutrient materials which inherently possess buffering properties, e.g. KQ and FKSH production media described herein below.

The preferred sources of carbon in the nutrient medium are carbohydrates such as glucose, xylose, galactose, glycerin, starch, dextrin, and the like. Other sources which may be included are maltose, rhamnose, raffinose, arabinose, mannose, salicin, sodium succinate, and the like.

The preferred sources of nitrogen are yeast extract, meat extract, peptone, gluten meal, cottonseed meal, soybean meal and other vegetable meals (partially or totally defatted), casein hydrolysates, soybean hydrolysates and yeast hydrolysates, corn steep liquor, dried yeast, wheat germ, feather meal, peanut powder, distilled solubles, etc., as well as inorganic and organic nitrogen compounds such as ammonium salts (e.g. ammonium nitrate, ammonium sulfate, ammonium phosphate, etc.,), urea, amino acids, and the like.

The carbon and nitrogen sources, though advantageously employed in combination, need not be used in their pure form, because less pure materials which contain traces of growth factors and considerable quantities of mineral nutrients, are also suitable for use. When desired, there may be added to the medium mineral salts such as sodium or calcium carbonate, sodium or potassium phosphate, sodium or potassium chloride, sodium or potassium iodide, magnesium salts, copper salts, cobalt salts, and the like. If necessary, especially when the culture medium foams seriously, a defoaming agent, such as liquid paraffin, fatty oil, plant oil, mineral oil or silicone may be added.

The preferred culturing/production medium for carrying out the fermentation is as follows:

The seed medium consisted of glucose 2.0% yeast extract 2.0%, Hy-Case SF 2.0%, $KN0_3$ 0.2%, $CaCl_2{\cdot}2H_20$ 0.002%, $ZnS0_4{\cdot}7H_20$ 0.001%, $MnS0_4{\cdot}H_20$ 0.0005°%, $FeS0_4{\cdot}7H_2$ 0 0.0025%, $MgS0_4{\cdot}7H_2$ 0 0.05%, NaCl 0.05%, and distilled $H_2$ 0 1.0 liter. The pH of the medium was adjusted to 7.0 with Na0H before autoclaving. Cultures were shaken at 27°C and 220 rpm for 42 hours.

Production cultures were initiated by adding 0.5 ml of seed culture to 15 ml of production medium in a 250 ml nonbaffled flask, incubated at 27°C, and shaken at 240 rpm. Production medium consisted of glucose 2.2%, glycerol 2.5%, corn steep liquor 1.0%, yeast extract 1.5%, $CaC0_3$ 0.025, lactic acid 0.2%, L-tyrosine 0.4%, morpholinopropanesulfonic acid 1.0%, and distilled water 1.0 liter.

The immunomycin analogs produced can be recovered from the culture medium by conventional means which are commonly used for the recovery of other known biologically active substances. Immunomycin and its analogs are found in the cultured mycelium and filtrate, and accordingly can be isolated and purified from the mycelium and the filtrate, which are obtained by filtering or centrifuging the cultured broth, by a conventional method such as concentration under reduced pressure, lyophilization, extraction with a conventional solvent, such as methanol and the like, pH adjustment, treatment with a conventional resin (e.g. anion or cation exchange resin, non-ionic adsorption resin, etc.), treatment with a conventional adsorbent (e.g. activated charcoal, silicic acid, silica gel, cellulose, alumina, etc.), crystallization, recrystallization, and the like. A preferred method is solvent extraction, particularly using methanol.

The preferred strain is a mutant strain of Streptomyces hygroscopicus subsp. ascomyceticus, Merck No. MA6678, ATCC No. 55087.

The broth from the fermentation exhibits positive immunosuppressive activity by the "T-cell proliferation assay" and possesses utility on this basis.

The immunomycin analogs obtained according to the fermentation processes as explained above can be isolated and purified in a conventional manner, for example, extraction, precipitation, fractional crystallization, recrystallization, chromatography, and the like. They can be used as immunosupressants according to the procedures known in the art.

Suitable salts of these homologs may include pharmaceutically acceptable salts, such as basic salts, for example, alkali metal salt (e.g. sodium salt, potassium salt, etc.), alkaline earth metal salt (e.g. calcium salt, magnesium salt, etc.), ammonium salt, amine salt (e.g. triethylamine salt, N-benzyl-N-methylamine salt, etc.) and other conventional organic salts.

It is to be noted that in the aforementioned fermentation reactions and the post-treatment of the fermentation mixture therein, the geometric isomer and/or stereoisomer(s) of analogs of immunomycin due to asymmetric carbon atom(s) or double bond(s) of the compound may occasionally be transformed into the other isomer(s), and such isomers are also included within the scope of the present invention.

The immunomycin analog of the present invention, possesses pharmacological activity such as immunosuppressive activity, antimicrobial activity, and the like, and therefore are useful for the treatment and prevention of the transplantation rejection of organs or tissues such as heart, kidney, liver, medulla ossium, skin, etc., graft-versus-host diseases by medulla ossium transplantation, autoimmune diseases such as rheumatoid arthritis, systemic lupus erythematosus, Hashimoto's thyroiditis, multiple sclerosis, myasthenia gravis, type I diabetes, uveitis, etc., infectious diseases caused by pathogenic microorganisms, and the like.

The pharmaceutical composition of this invention can be used in the form of a pharmaceutical preparation, for example, in solid, semisolid or liquid form, which contains the immunomycin analog of the present invention, as an active ingredient, in admixture with an organic or inorganic carrier or excipient suitable for external, enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The active object compound is included in the pharmaceutical composition in an amount sufficient to produce the desired effect upon the process or condition of diseases.

For applying this composition to a human, it is preferable to apply if by parenteral or enteral administration. While the dosage of therapeutically effective amount of the immunomycin analog varies from, and also depends upon the age and condition of each individual patient to be treated, a daily dose (calculated on the basis of a 70 kg man) of about 0.01-1000 mg, preferably 0.1-500 mg and more preferably 0.5-100 mg, of the active ingredient is generally given for treating diseases, and an average single dose of about 0.5 mg, 1 mg, 5 mg, 10 mg, 50 mg, 100 mg, 250 mg and 500 mg is generally administered.

The following examples are given for the purpose of illustrating the present invention and should not be construed as being limitations on the scope or spirit of the instant invention.

EXAMPLE 1

MICROORGANISM AND CULTTURE CONDITIONS

Streptomyces hygroscopicus, subsp. ascomyceticus was originally obtained from the American Type Culture Collection (No. 14891). It was deposited in the Merck culture collection and designated Merck No. MA6475. A strain of Merck No. MA6475 which produces increased quantities of the following immunosuppressants: immunomycin, the ethyl analog at C21 and compound B, the methyl analog at C21 isolated from a mutated population of Merck No. MA6475, ATCC No. 14891 and designated Merck No. MA6678, ATCC No. 55087.

EXAMPLE 2

FERMENTATION

Cultivation was begun by adding 1.0 ml of frozen vegetative cells, preserved in 10% glycerol, to 50 ml of seed medium in a 250 ml baffled erylenmeyer. The seed medium consisted of glucose 2.0% yeast extract 2.0%, Hy-Case SF 2.0%, $KN0_3$ 0.2%, $CaCl_2 \cdot 2H_20$ 0.002%, $ZnS0_4 \cdot 7H_2$ 0 0.001%, $MnS0_4 \cdot H_2$ 0 0.0005%, $FeS0_4 \cdot 7H_2$ 0 0.0025%, $MgS0_4 \cdot 7H_20$ 0.05%, NaCl 0.05%, and distilled $H_2$ 0 1.0 liter. The pH of the medium was adjusted to 7.0 with Na0H before autoclaving. Cultures were shaken at 27°C and 220 rpm for 42 hours.

Production cultures were initiated by adding 0.5 ml of seed culture to 15 ml of production medium in a 250 ml nonbaffled flask, incubated at 27°C, and shaken at 240 rpm. Production medium consisted of glucose 2.2%, glycerol 2.5%, corn steep liquor 1.0%, yeast extract 1.5%, $CaC0_3$ 0.025, lactic acid 0.2%, L-tyrosine 0.4%, morpholinopropanesulfonic acid 1.0%, and distilled water 1.0 liter. The pH of the medium was adjusted to 6.8 with NaOH before autoclaving. A neutral solution of the fatty acid or related substrate for the purpose of directing the fermentation was added to the fermentation at some time during the first 54 hours of growth either in one addition or in several smaller portions to give a final concentration of the substrate of 10 to 30 mM. Fermentations were harvested at 72 or 96 hours.

EXAMPLE 3

Four 25mm tubes containing 3.0 ml of production media were each inoculated with 0.1 ml of a well grown seed of MA6678. To two tubes was added 0.1 ml of a neutralized solution of valeric acid (300 mN) at 26, 48,

and 54 hours. The tubes were harvested at 72 hours. The contents of the tubes receiving valerate were combined together as were the control tubes which received no addition. A 3.0 ml portion of each whole broth sample was extracted with an equal volume of methanol. After evaporation of the methanol, the remaining aqueous solutions were extracted with equal volumes of ethyl acetate. The ethyl acetate was evaporated and the residues dissolved in 0.25 ml of MeOH. A 50 µl aliquot of each MeOH solution was fractionated using the analytical HPLC system run in acetonitrile/0.1% $H_3P0_4$ (42:58, V/V). One minute fractions were collected starting at 10 minutes and continuing until 25 minutes. To each fraction was added 50 µl of a 0.2M MES solution (pH 7.4). The fractions were evaporated to dryness, and submitted for IL-2 assay. Under these HPLC conditions, retention times for the immunomycin-like compounds were: compound B at 12:97 minutes, immunomycin at 15:31 minutes, and a new peak, (compound C) at 19:43 minutes.

EXAMPLE 4

Two hundred 250 ml flasks containing 15 ml of production medium were each inoculated with 0.5 ml of a well grown seed culture of MA6678. At 24 and 48 hours a 0.5 ml addition was made to each flask of a 300 mM solution of n-valeric acid which had been pH adjusted to 6.4 with NaOH and filter sterilized. At 72 hours the contents of the flasks were pooled and centrifuged. The mycelium and supernatant were extracted with MeOH and ethyl acetate as described in the ISOLATION section above. Evaporation of the combined ethyl acetate extracts yielded an oily brown residue. An HPLC assay of the residue dissolved in 5.5 ml of MeOH indicated the presence of 55.3 mg of compound B, 217.6 mg of immunomycin, and 4.3 mg of compound C. Separation of compound C from immunomycin was accomplished by two purification runs through a Rainin semi-preparative HPLC system. This afforded 542 µg of compound C which was then analyzed by proton NMR and mass spectrometry. In addition, an $IC_{50}$ determination as pait of the IL-2 assay was determined.

EXAMPLE 5

ISOLATION OF IMMUNOMYCIN AND ANALOGUES

The whole broth was centrifuged and the mycelium extracted with methanol (1:1, W/V). The methanol was removed using a rotary evaporator and the resulting aqueous solution was extracted with an equal volume of ethyl acetate. The supernatant broth was extracted with an equal volume of ethyl acetate. The supernatant broth was extracted with ethyl acetate (2:1, V/V), and the ethyl acetate extracts from mycelium and supernatant combined, and dried over anhydrous sodium sulfate. The ethyl acetate was removed under vacuum and the residue dissolved in methanol.

CHROMATOGRAHY

Separation of analogues in the presence of immunomycin was accomplished by chromatographing the material once or repeatedly on a semi-preparative Rainin Dynamax HPLC column. Titers of immunomycin and its analogs were determined by analytical HPLC using a Whatman Partisil-5 0DS-3 column (4.6 mm x 25 mm) operated at 60°C and eluted isocratically with a mobile phase consisting of acetonitrile/ 0.1% $H_3P0_4$ (65:35) run at 1.0 ml/minute. Immunomycin and its analogs were detected at 205 nm. Semi-preparative HPLC was accomplished using a Rainin Dymamax column (10mm x 25cm) operated at 50°C and eluted isocratically with acetonitrile/ 0.1% $H_3P0_4$ (55:45) run at 4.0 ml/minute. Detection was at 205 nm.

EXAMPLE 6

FRACTIONATION FOR IL-2 ASSAY

Biological activity of peaks observed in the HPLC profiles were determined by collecting 0.5 or 1.0 minute fractions of a sample run on the analytical HPLC. The pH of each sample was adjusted by adding 50 µl of a 0.2 M solution of morpholinoethanesulfonic acid (MES, pH 7.4) to each 1.0 ml fraction. Fractions were then evaporated to dryness using a Speed Vac Concentrator (Savant) and redissolved in ethanol for IL-2 assay.

EXAMPLE 7

T-CELL PROLIFERATION ASSAY

1. Sample Preparation

The purified fraction containing compound C, as prepared by HPLC above, was dissolved in absolute ethanol at a final concentration of 100 μg/ml.

2. Assay

Spleens from C57B1/6 mice were taken under sterile conditions and gently dissociated in ice-cold RPMI 1640 culture medium (GIBCO, Grand Island, N. Y.) supplemented with 10% heat-inactivated fetal calf serum (GIBCO). Cells were pelleted by centrifugation at 1500 rpm for 8 minutes. Contaminating red cells were removed by treating the pellet with ammonium chloride lysing buffer (GIBCO) for 2 minutes at 4°C. Cold medium was added and cells were again centrifuged at 1500 rpm for 8 minutes. T lymphocytes were then isolated by separation of the cell suspension on nylon wool columns as follows: Nylon wool columns were prepared by packing approximately 4 grams of washed and dried nylon wool into 20 ml plastic syringes. The columns were sterilized by autoclaving at 250°F for 30 minutes. Nylon wool columns were wetted with warm (37°C) culture medium and rinsed with the same medium. Washed spleen cells resuspended in warm medium were slowly applied to the nylon wool. The columns were then incubated in an upright position at 37°C for 1 hour. Non-adherent G lymphocytes were eluted from the columns with warm culture medium and the cell suspensions were spun as above.

Purified T lymphocytes were resuspended at 2.5 x $10^5$ cells/ml in complete culture medium composed of RPMI 1640 medium with 10% heat-inactivated fetal calf serum, 100 mM gultamine, 1mM sodium pyruvate, 2 x $10^{-5}$M 2-mercaptoethanol and 50 μg/ml gentamycin. Ionomycin was added at 250 ng/ml and PMA at 10 ng/ml. The cell suspension was immediately distributed into 96 well flat-bottom microculture plates (Costar) at 200 μl/well. The control, being the medium without test drug, and various below-indicated dilutions of the sample (above-described purified compound C) to be tested were then added in triplicate wells at 20 μl/well. compound A was used as a standard. The culture plates were then incubated at 37°C in a humidified atmosphere of 5% $CO_2$ -95% air for 44 hours. The proliferation of T lymphocytes was assessed by measurement of tritiated thymidine incorporation. After 44 hours of culturing, the cells were pulse-labelled with 2 μCi/well of tritiated thymidine (NEN, Cambridge, MA). After another 4 hours of incubation, cultures were harvested on glass fiber filters using a multiple sample harvester. Radioactivity of filter discs corresponding to individual wells as measured by standard liquid scintillation counting methods (Betacounter). Mean counts per minute of replicate wells were calculated and the results expressed as percent inhibition of tritiated thymidine uptake (profileration) as follows

$$\text{Inhibition} = 100 - \frac{\text{Mean cpm sample tested X 100}}{\text{Mean cpm control medium}}.$$

The results of % inhibition at various concentrations of compound C are presented in Table 1:

TABLE 1

Inhibition of T-Cell Proliferation by Active Fraction (containing Compound C)

| Concentration of Compound C (ng/ml) | % Inhibition |
|---|---|
| 10.0 | 99.9 |
| 6.6 | 98.3 |
| 4.4 | 97.9 |
| 2.9 | 96.6 |
| 1.9 | 94.0 |
| 1.3 | 88.9 |
| 0.87 | 45.2 |
| 0.58 | 14.8 |
| 0.39 | 0.0 |

Notes:

1. Mouse T cell cultures were pulsed with $^{3}$H-thymidine for 4 hours prior to harvesting at 48 hours.
2. Standard L-679,934 (10 ng/ml) gave 99% inhibition.
3. IC-50=0.82 ng/ml = 1.02 nM for fraction 510.
4. Inhibition of T-cell proliferation by Fraction 510 was reversed by the addition of 50 units/ml of recombinant human IL-2 at the initiation of culture.

EXAMPLE 8

MASS SPECTROMETRY

A sample of compound C was analyzed by FAB mass spectrometry using a MAT-731 mass spectrometer operated at 8 kv. The matrix employed was a 5:1 mixture of dithiothreitol/dithioerythritol which contained a trace amount of lithium acetate. The sample was ionized using a xenon fab gun at 8 kv. A pseudo-molecular ion was observed at m/z = 812 (M + Li) corresponding to a nominal molecular weight of 805 amu. A matrix adduct ion (M + Li + matrix) was observed at m/z = 966 (805 + 7 + 154).

EXAMPLE 9

$^{1}$H NMR SPECTROSCOPY

The proton NMR spectra were obtained in $CDCl_3$ using a Varian XL-400 spectrometer. Chemical shifts are expressed as parts per million relative to $CHCl_3$ set at 7.26 ppm. Figure 1 is the $^{1}$H NMR of Compound C prepared synthetically. Figure 2 is the $^{1}$H NMR of Compound C prepared by directed biosynthesis.

## Claims

**1.** A process for the preparation of a compound of Formula I

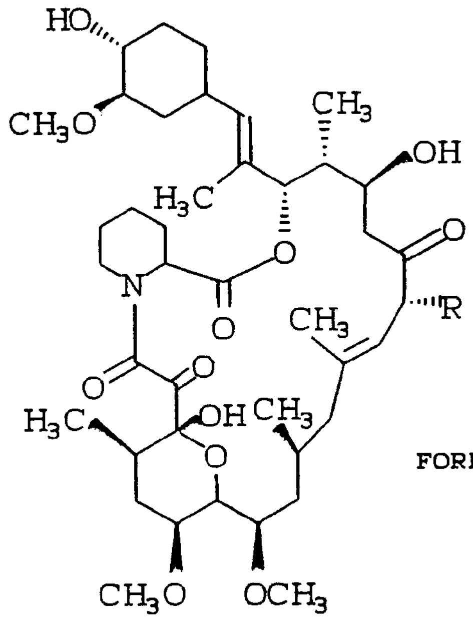

FORMULA I

wherein R is propyl which comprises fermenting an immunomycin producing strain such as Streptomyces hygroscopicus subsp. ascomyceticus (Merck No. MA6475, ATCC No. 14891) or a mutant strain obtained therefrom, such as Merck No. MA6678, ATCC No. 55087, in aqueous nutrient medium containing an assimilable source of carbon, an assimilable source of nitrogen and inorganic salts under aerobic conditions with a valeric acid or a salt thereof.

2. The process of Claim 1 wherein the strain is a mutant strain of Streptomyces hydroscopicus subsp. ascomyceticus (ATTC 14891).

3. The process of Claim 1, wherein the directed biosynthesis employs the mutant strain of Streptomyces hygroscopicus subsp. ascomyceticus identified as Merck No. MA6678, ATCC No. 55087,

4. The process of Claim 3, wherein the fermentation is conducted at a temperature between 24 and 30°C and a pH range from about 6.0 to 7.5, with agitation supplied by a rotary shaker operating between 150 and 300 rpm.

5. The process of Claim 4, wherein the nutrient medium contains about 0.5 to 5.0% by weight of carbon source and about 0.2 to 6.0% by weight of nitrogen.

6. The process of Claim 5, wherein the fermentation is carried out for 1 to 8 days.

7. The process of Claim 6, wherein the strain producing the immunomycin analogs is a mutant strain of Streptomyces hygroscopicus subsp. ascomyceticus, ATCC No. 14891.

8. A process for the preparation of a seed culture for fermentative production of the compound of Formula I wherein R is propyl, which comprises a fermentation of the mutant strain Merck No. MA6678, ATCC No. 55087: 1) using 1.0 ml frozen vegetative cells preserved in 10% glycerol to 50 ml of a seed medium which consists of: glucose 2.0% yeast extract 2.0%, Hy-Case SF 2.0%, $KN0_3$ 0.2%, $CaCl_2{\cdot}2H_20$ 0.002%, $ZnS0_4{\cdot}7H_20$ 0.001%, $MnS0_4{\cdot}H_20$ 0.0005%, $FeS0_4{\cdot}7H_2$ 0 0.0025%, $MgS0_4{\cdot}7H_2$ 0 0.05%, NaCl 0.05%, and 1.0 L distilled $H_20$; 2) adjusting the pH of the medium to 7.0 with NaOH; 3) autoclaving; and 4) shaking the tubes at 230 rpm for 42 hours at 27°C.

9. A process for the preparation of a compound of Formula I wherein R is propyl, which comprises 1) the mixing of 0.5 ml of the seed culture of Claim 8 with 15 ml of production medium consisting of: glucose 2.2%, glycerol 2.5%, corn steep liquor 1.0%, yeast extract 1.5%, $CaC0_3$ 0.025, lactic acid 0.2%, L-tyrosine 0.4%, morpholinopropanesulfonic acid 1.0%, and 1.0L distilled water; 2) incubating at 27°C with agitation at 240 rpm; 3) adjusting pH to 6.8; 4) autoclaving; 5) adding valeric acid (pentanoic acid) during the first

54 hours; and 6) harvesting at 3-4 days.

**10.** A novel culture strain Merck No. 6678, ATCC No. 55087.

FIG. 1

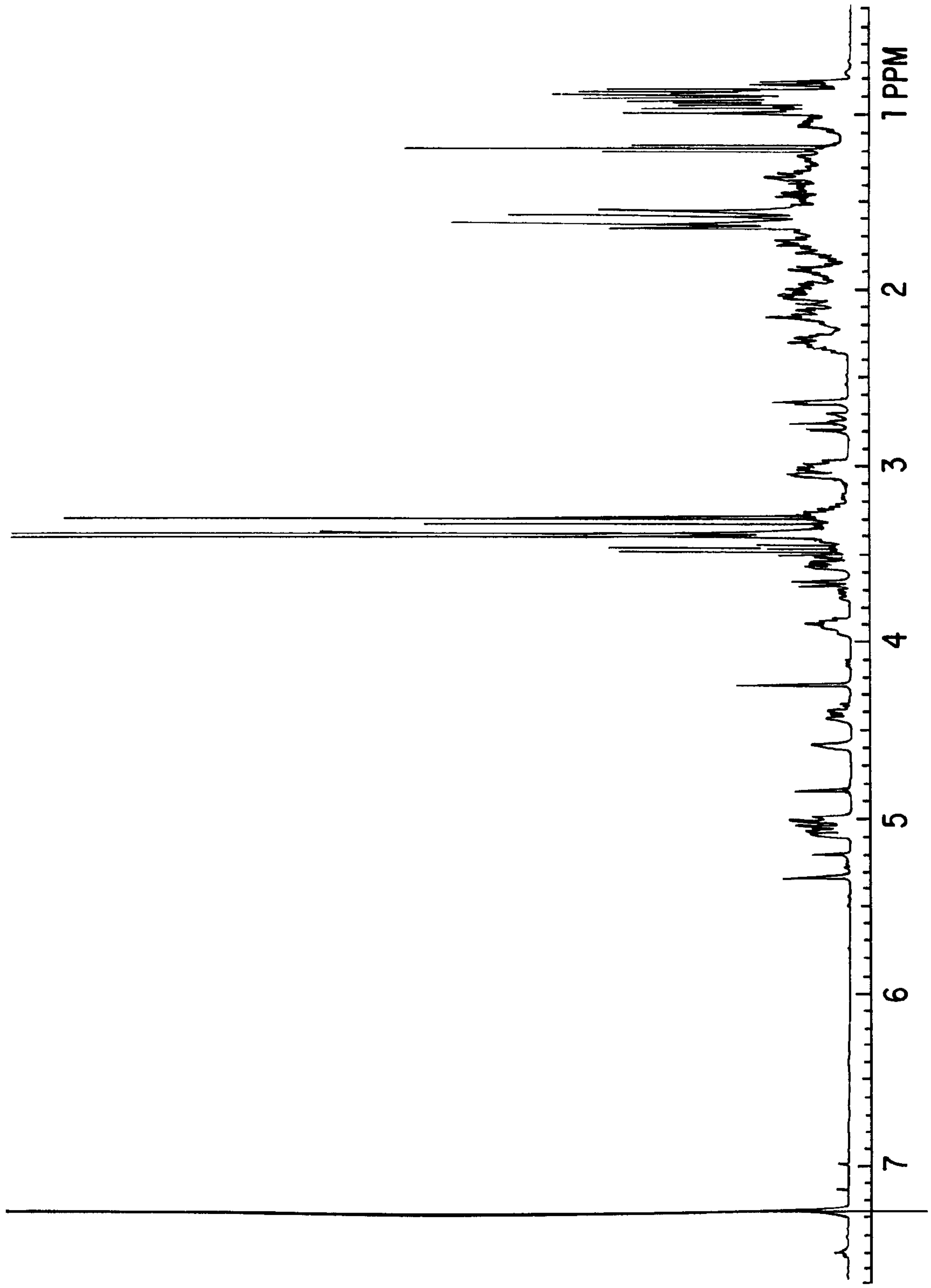

FIG. 2

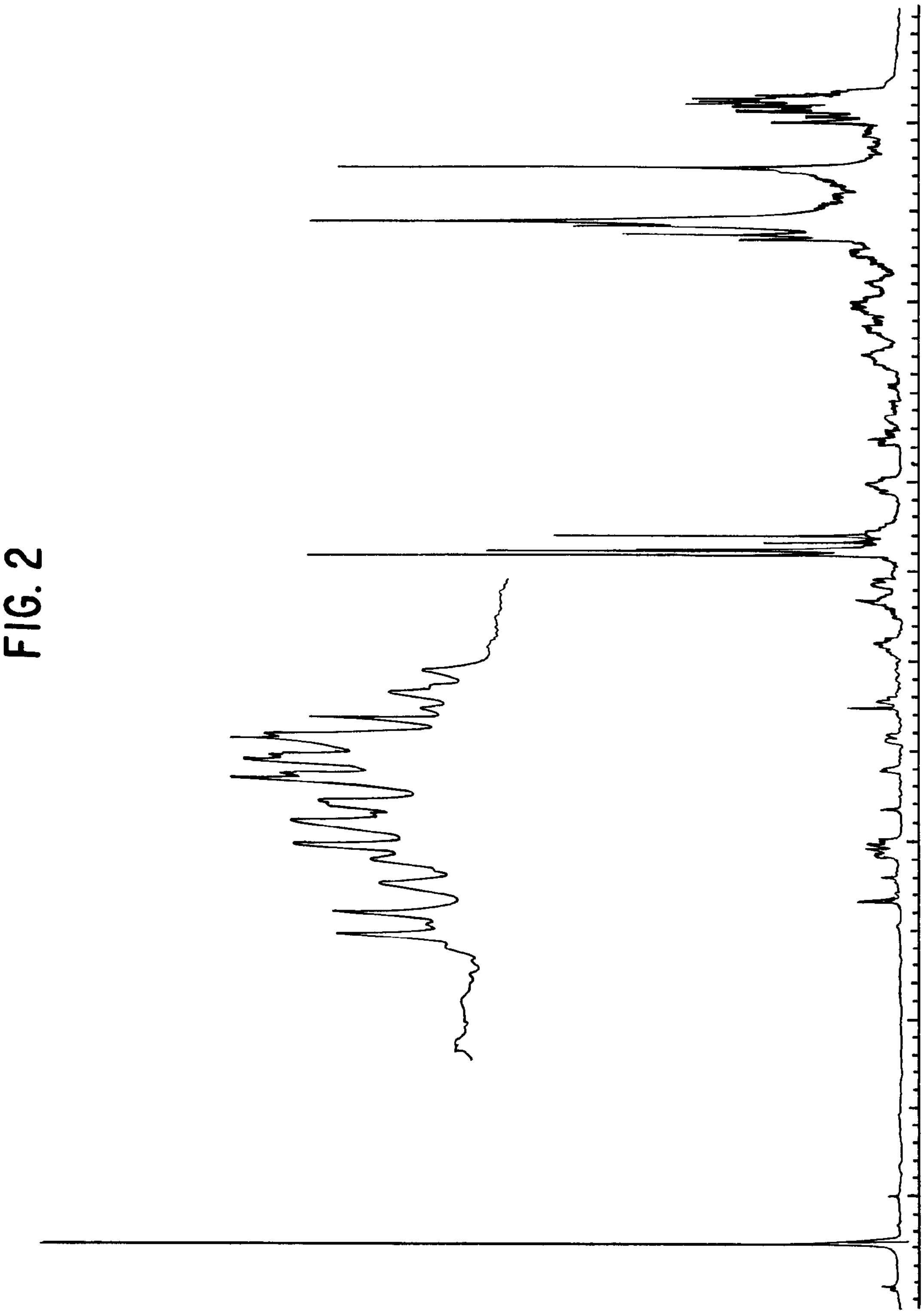

European Patent Office

# EUROPEAN SEARCH REPORT

Application Number

EP 91 30 9916

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 323 865 (MERCK AND CO. INC.)<br>* claims; example 1 *<br>--- | 1 | C 07 H 19/01<br>C 12 N 1/20<br>C 12 P 19/26 //<br>(C 12 P 19/26<br>C 12 R 1:55 )<br>(C 12 N 1/20<br>C 12 R 1:55 ) |
| A | EP-A-0 388 153 (MERCK AND CO. INC.)<br>* claims; example 1 *<br>--- | 1 | |
| A | EP-A-0 388 152 (MERCK AND CO. INC.)<br>* claims; example 1 *<br>--- | 1 | |
| A | EP-A-0 323 042 (FISONS PLC)<br>* claims; examples 7-12 *<br>--- | 1 | |
| A | EP-A-0 184 162 (FUJISAWA PHARMACEUTICAL CO.,LTD.)<br>* claims; example 21 *<br>----- | 1 | |
| | | | TECHNICAL FIELDS SEARCHED (Int. Cl.5) |
| | | | C 07 H<br>C 12 P<br>C 12 N |

The present search report has been drawn up for all claims

| Place of search | Date of completion of the search | Examiner |
|---|---|---|
| THE HAGUE | 14-02-1992 | DAY G.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)